# EUROPEAN PATENT APPLICATION

(11) **EP 1 825 858 A1**
(43) Date of publication of application: **29.08.2007**
(21) Application number: 05806022.9
(22) Date of filing: 14.11.2005
(51) Int. Cl.: A61K 33/30, A61K 31/045, A61P 19/00, A61P 19/08, A61P 19/10, A23K 1/16, A23K 1/175, A23L 1/30, A23L 1/304, A23L 2/52

(54) **COMPOSITION FOR PROMOTING OSTEOGENESIS AND INCREASING BONE MINERAL CONTENT**

(30) Priority: 16.11.2004 JP 2004332048
(71) Applicant: Yamaguchi, Masayoshi, Shizuoka-shi, Shizuoka 420-0913 (JP)
(72) Inventor: Yamaguchi, Masayoshi, Shizuoka-shi, Shizuoka 420-0913 (JP)
(74) Representative: Evans, Jacqueline Gail Victoria
(86) International application number: PCT/JP2005/020887
(87) International publication number: WO 2006/054530

(57) **Abstract**

It is to provide a composition for promoting osteogenesis and increasing bone mineral content with a function of (promoting) osteogenesis and increasing bone mineral content, having a significant effect, which promotes actively osteogenesis to prevent/treat bone diseases, a preventative/therapeutic agent for bone diseases, a functional food or food material for preventing/treating bone diseases, and feed composition. A composition for promoting osteogenesis and increasing bone mineral content and a preventative/therapeutic agent for bone diseases comprising β-cryptoxanthin and zinc compound at a concentration that does not exhibit a significant effect of increasing bone calcium level when used separately, as active ingredients are prepared. Further, β-cryptoxanthin and zinc compound are added/compounded to a food or food material, or feed, at a concentration that does not exhibit a significant increasing effect of bone calcium level, when used separately.

## Description

### Technical Field

The present invention relates to a composition for promoting osteogenesis and increasing bone mineral content comprising β-cryptoxanthin and zinc compound as active ingredients; a preventative/therapeutic agent for bone diseases such as osteoporosis; a functional food or food material, or feed composition for preventing/treating bone diseases such as osteoporosis added with β-cryptoxanthin and zinc compound.

### Background Art

It is considered that various bone diseases occur because, for example, calcium level of bones is decreased by abnormal bone metabolism or insufficient osteogenesis. Typical bone diseases are, for example, fracture, osteomalacia, osteopenia, osteoporosis, back pain and low back pain. In those bone diseases, osteoporosis has a pathology caused by the following reasons: the bone mass is decreased as the balance of bone resorption and bone formation is lost by aging and, accordingly, the bone resorption is relatively increased to reduce the bone mass. As a result, the bone strength is decreased by the change in the fine structure of bones to easily cause a fracture. Particularly in female, the bone mass is rapidly decreased after menopause, oophorectomy, etc. Osteoporosis not only causes fractures or sharp pain, but it also makes the patients bedridden, particularly in cases of elderly people. Under these circumstances, an effective cure is demanded for improving the quality of life in an aging society. Because it is difficult to cure patients with osteoporosis after the onset, the following points are now fully recognized: it is important to prevent this disease and it is also indispensable to start increasing the bone mass from juvenile period. In addition, it is essential that nutrients required for the formation of bones and foods accelerating the formation of them must be taken everyday. As foods for strengthening bones, calcium, magnesium and vitamin D are mainly used nowadays. Casein phosphopeptide or the like which promotes absorption of calcium through intestinal tracts is also used.

As therapeutic agents for bone diseases such as osteoporosis, active vitamin D₃, female sex hormone (estrogen), calcitonin and ipriflavones are clinically used. Recently, an anti-osteoporosis agent having an effect of polyisoprenoid derivatives typified by vitamin K₂ for inhibiting the formation of osteoclasts has been proposed (see for example patent document 1). Further, the followings are known: a bone reinforcing agent containing casein phosphopeptide and genistein as active ingredients (see for example patent document 2); a composition for promoting osteogenesis, which is effective against osteoporosis and which contains saponin, daidzin, daidzein, genistin and genistein as main active ingredients (see for example patent document 3); a composition for increasing bone mass, which is effective against osteoporosis and which contains Japanese horseradish extract as an active ingredient (see for example patent document 4); and a composition for promoting osteogenesis and preventing reduction of bone mineral content, which contains isoflavone as a main active ingredient (see for example patent document 5) .

On the other hand, β-cryptoxanthin (molecular weight: 552) is known as a carotenoid soluble in ethanol, which is contained in citrus fruits, particularly in Satsuma oranges in an amount of 1 to 2 mg per fruit. β-cryptoxanthin has characteristic properties of provitamin A, as a nutrient component. In addition, in a recent investigation of anticancer substances, it was found that β-cryptoxanthin has an anticancer effect stronger than that of β-carotene which is a carotenoid contained in green and yellow vegetables such as carrots and, therefore, β-cryptoxanthin is drawing a lot of attention (see for example non-patent document 1). As β-cryptoxanthin is an important component inhibiting carcinogenesis, the followings are proposed in order to contribute to the development of citrus fruits or citrus prepared foods in which β-cryptoxanthin is enhanced: production of citrus fruits of a high quality having a a content comparable to that of the Satsuma oranges, or isolation of genes synthesizing β-cryptoxanthin (see for example patent document 6, patent document 7), a method for producing carotenes such as β-cryptoxanthin by using bacteria belonging to the genus Paracoccus (see for example patent document 8); a method for producing β-cryptoxanthin of a high purity from a precipitant of raw material obtained by pressing orange juice (see for example patent document 9). Moreover, the present inventors have recently reported an osteogenesis promoter comprising β-cryptoxanthin as an active ingredient, or that β-cryptoxanthin promotes osteogenesis at a concentration of 10⁻⁸ to 10⁻⁶ M with a 48 h culture in a bone tissue culture system, to increase bone calcium (see for example patent document 10, non-patent document 2).

On the other hand, concerning osteogenesis, it has been reported that when zinc compound which is a microelement, promotes osteogenesis and suppresses bone absorption, there was a restoration effect in various animal models having experimental bone pathology for bone formation (see for example non-patent document 3). Further, the present inventors have reported that zinc promotes synthesis of bone protein at a concentration of 10⁻⁴ M or more, and exhibits an effect of increasing bone calcium according to osteogenesis enhancement (see for example non-patent document 4). Furthermore, it is known to use isoflavone and zinc salt (see for example patent document 11), isoflavone and organic zinc (see for example patent document 12) as an osteogenesis promoter, and to use vitamin K and zinc (see for example patent document 13) as an anti-osteoporosis composition.

However, the synergistic increase of bone calcium level by using β-cryptoxanthin and zinc in combination is completely unknown, and the synergistic effect that is observed by using β-cryptoxanthin and zinc in combination in an amount that does not exhibit a bone calcium increasing effect when used separately is not known at all.

Patent document 1: Japanese Laid-Open Patent Application No. 7-215849
Patent document 2: Japanese Laid-Open Patent Application No. 2001-302539
Patent document 3: Japanese Laid-Open Patent Application No. 2000-191526
Patent document 4: Japanese Laid-Open Patent Application No.10-279492
Patent document 5: Japanese Laid-Open Patent Application No. 10-114653
Patent document 6: Japanese Laid-Open Patent Application No. 11-155577
Patent document 7: Japanese Laid-Open Patent Application No. 11-46770
Patent document 8: Published Japanese translation of PCT international publication No. 2001-512030
Patent document 9: Japanese Laid-Open Patent Application No. 2000-136181
Patent document 10: WO 2004/037236
Patent document 11: Japanese Laid-Open Patent Application No. 10-114653
Patent document 12: Japanese Laid-Open Patent Application No. 11-346716
Patent document 13: Japanese Laid-Open Patent Application No. 10-36256
Non-patent document 1: Biological & Pharmaceutical Bulletin, 18, 2, 227, 1995
Non-patent document 2: Molecular and Cellular Biochemistry, 258, p.137-144, 2004
Non-patent document 3: Annual Report of Sugiyama Sangyo Kagaku Research Institute (1996), Sugiyama Sangyo Kagaku Research Institute, Jan. 30, 1997, p.85-93
Non-patent document 4: Biochemical Pharmacology, 36, p 4007-4012, 1987

### Disclosure of the Invention

### Object to be solved by the Invention

It was reported that some therapeutic agents now approved in Japan for bone diseases typified by osteoporosis are bone resorption-inhibitors (inhibiting the solution of bones) and also that only statin, which is a mevalonic acid synthetic inhibitor, has an osteogenesis promoting effect. However, this finding is only on a gene level and, in fact, the osteogenesis promoting effect of statin was weak. Further, in Europe and United States, transgenic human parathyroid hormone is used, while its use is limited from the point of view of side effects. The object of the present invention is to provide food and drink, pharmaceuticals, or feed useful for preventing/treating bone diseases such as osteoporosis having an effect of promoting osteogenesis, and increasing salt mineral content, exhibiting a significant effect for preventing/treating bones diseases, by promoting actively osteogenesis.

### Means to Solve the Object

The inventors have reported that β-cryptoxanthin, which is contained in a large amount in peel and sarcocarp of Satsuma orange, has an osteogenesis-promoting effect and effect of preventing/treating bone diseases (see for example non-patent document 3). Namely, the inventors cultured diaphysis and metaphysis tissues of a femur in a culture medium containing β-cryptoxanthin, then measured the calcium level in bone tissues, the amount of the expressed bone calcification accelerating enzyme and DNA level which is an index of cell count in bone tissues, and confirmed a significant increase in all of the cases. In the experiments, the present inventors have found that β-cryptoxanthin accelerates the synthesis of protein in the cancellous bone (metaphysis tissues) and cortical bone (diaphysis tissues) in femur tissues to promote osteogenesis. The effective concentration of β-cryptoxanthin for exhibiting an effect of promoting osteogenesis and increasing bone calcium level was a concentration of 10⁻⁸ - 10⁻⁶ M in a 48-hour culture system of a bone tissue culture system. However, even β-cryptoxanthin can be obtained by various methods including a method for separating and extracting from citrus, especially Satsuma orange, a genetic method or a microbiological culture method, it cannot be said that it can be obtained at a low cost. Thus a use in combination with other compounds exhibiting similar effect at a lowest concentration was considered.

On the other hand, as bone tissues contain a large amount of zinc, involvement of zinc with osteogenesis has been investigated recently, and the osteogenesis effect of zinc has been confirmed. Zinc is contained widely in foods, while the average intake of Japanese is 6 - 12 mg, which is insufficient compared to the recommended amount (adult 15 mg, Fourth revised Table of food component 1996, p 441, Kagawa Nutrition University Publishing Division). Therefore, the bone effect of zinc alone is not sufficient at a regular diet. Taking regard of this situation, the present inventors selected and investigated the above mentioned zinc compound as a compound to be used in combination with β-cryptoxanthin, they surprisingly found out that a synergetic effect was exhibited with a concentration with which neither of compounds exhibit osteogenesis promoting effect and effect for increasing bone mineral content when used separately. The present invention has been thus completed.

Specifically, the present invention relates to (1) a composition for promoting osteogenesis and increasing bone mineral content comprising β-cryptoxanthin and zinc compound as active ingredients; (2) the composition for promoting osteogenesis and increasing bone mineral content according to (1), wherein β-cryptoxanthin and zinc compound are contained at a concentration that does not exhibit an effect of increasing calcium level in bone tissues when used independently; (3) a preventative/therapeutic agent for bone diseases comprising β-cryptoxanthin and zinc compound as active ingredients.

Further, the present invention relates to (4) the preventative/therapeutic agent for bone diseases according to (3), wherein β-cryptoxanthin and zinc compound are contained at a concentration that does not exhibit an effect of increasing calcium level in bone tissue when used independently; (5) a preventative/therapeutic agent for bone diseases according to (4), wherein the bone disease is osteoporosis; (6) a functional food or food material for preventing/treating bone diseases, wherein β-cryptoxanthin and zinc compound are added; (7) the functional food or food material for preventing/treating bone diseases according to (6), wherein β-cryptoxanthin and zinc compound are contained at a concentration that does not exhibit an effect of increasing calcium level in bone tissue when used independently.

Furthermore, the present invention relates to (8) a food and drink according to (7), wherein the bone disease is osteoporosis; (9) a feed composition wherein β-cryptoxanthin and zinc compound are compounded; (10) the feed composition according to (9), wherein β-cryptoxanthin and zinc compound are contained at a concentration that does not exhibit an effect of increasing calcium level in bone tissues when used independently.

### Brief Description of Drawings

[Fig. 1] It is a set of graphs showing the measurement results of calcium level in bone tissues (diaphysis and metaphysic parts) in a bone tissue culture system.
[Fig. 2] It is a set of graphs showing the measurement results of calcium level in bone tissues (diaphysis and metaphysic parts) in a bone tissue culture system.
[Fig. 3] It is a set of graphs showing the measurement results of the alkaline phosphatase activity in bone tissues (diaphysis and metaphysis parts) in a bone tissue culture system.
[Fig. 4] It is a set of graphs showing the measurement results of the DNA level in bone tissues (diaphysis and metaphysis parts) in a bone tissue culture system.
[Fig. 5] It is a set of graphs showing the measurement results of the calcium level in bone tissues (diaphysis and metaphysis parts) by oral administration.
[Fig. 6] It is a set of graphs showing the measurement results of the alkaline phosphatase activity in bone tissues (diaphysis and metaphysis parts) by oral administration.
[Fig. 7] It is a set of graphs showing the measurement results of the DNA level in bone tissues (diaphysis and metaphysis parts) by oral administration.

### Best Mode of Carrying Out the Invention

A composition for promoting osteogenesis and increasing bone mineral content of the present invention is not particularly limited as long as it comprises β-cryptoxanthin and zinc compound as active ingredients. Further, a preventative/therapeutic agent for bone diseases of the present invention is not particularly limited as long as it comprises β-cryptoxanthin and zinc compound as active ingredients. A functional food or food material for preventing/treating bone diseases of the present invention is not particularly limited as long as it is a food or food material having a function for preventing/treating bone diseases wherein β-cryptoxanthin and zinc compound are added. A feed composition of the present invention can be any one compounded with β-cryptoxanthin and zinc compound. The above bone diseases include, for example, bone fractures, osteomalacia, osteopenia, osteoporosis and back pain and low back pain. In particular, osteoporosis such as postmenopausal osteoporosis, estrogen-deficiency osteoporosis, senile osteoporosis and steroid-induced osteoporosis, as well as metabolic bone diseases such as osteomalacia can be preferably exemplified. The expression "for preventing/treating bone diseases" in the above-mentioned food or food material means, for example, that there is an indication that it is effective for preventing or treating bone diseases on the package or the attached manual of the food or food material.

As β-cryptoxanthin of the present invention, β-cryptoxanthin-containing material can be used advantageously besides β-cryptoxanthin. A method for manufacturing β-cryptoxanthin is not particularly limited, including known methods such as a method to extract and produce from citrus, a method for using genes encoding β-cryptoxanthin producing enzyme, and a method to obtain β-cryptoxanthin by culturing microbes producing the same. It is preferred to use Satsuma oranges containing β-cryptoxanthin in a high amount of 1 - 2 mg per orange, as a resource. Among Satsuma oranges, it is preferred to use Satsuma oranges containing β-cryptoxanthin in a high amount, such as Sugiyama Onshu containing approximately 8 mg of β-cryptoxanthin per 100 g of pericarp (exocarp), and 1 mg per 100 g of juice, or a cultivar containing β-cryptoxanthin in a high amount produced by mating with Satsuma Oranges. Further, in the present invention, a β-cryptoxanthin-containing material is a material mixed with β-cryptoxanthin in which β-cryptoxanthin content has been increased artificially. For example, a method for obtaining β-cryptoxanthin-containing material by treating Satsuma Oranges is not particularly limited, and can be obtained by known methods, such as concentration or extraction treatment.

A zinc compound of the present invention is not particularly limited, and inorganic zinc such as zinc, zinc sulfate, zinc chloride, chelating compounds of zinc gluconate and zinc, as well as natural salt produced from seawater, agents enhancing zinc concentration in foods such as zinc binding protein or peptides, foods containing high concentration of zinc, for example dried yeast, dried liver, snapping turtles, shark cartilage, Denshichi Ninjin, and oysters can be used directly as a zinc resource.

It is preferred that a composition for promoting osteogenesis and increasing bone mineral content, a preventative/therapeutic agent for bone diseases, a functional food or food material for preventing/treating bone diseases, a compound of β-cryptoxanthin and zinc in a feed composition of the present invention, are contained, added, compounded in a concentration that does not exhibit a function of increasing calcium level in bone tissues when used separately, and in a concentration that exhibits an effect of increasing calcium level in bone tissues, when used in combination. Specifically, a composition for promoting osteogenesis or increasing bone mineral content, or a preventative/therapeutic agent for bone disease, can be compounded so that the intake level of β-cryptoxanthin becomes 1 - 100 mg, preferably 1 to 50 mg per day, and that of zinc compound, converted into zinc, becomes 10 - 1000 mg, preferably 10 - 100 mg per day. In a functional food or food material, or feed composition for preventing/treating bone diseases, β-cryptoxanthin can be added or compounded in an amount of 0.1 - 20 mg, preferably 1 - 10 mg per 1 kg of solid content, and zinc compound can be added or compounded in an amount of 1 - 30 mg, preferably 10 - 20 mg, converted into zinc.

A composition for promoting osteogenesis or increasing bone mineral content comprising β-cryptoxanthin and zinc compound of the present invention as active ingredients can be used advantageously as a preventative/therapeutic agent for bone diseases of the present invention. When preparing a preventative/therapeutic agent for bone diseases of the present invention as a medical product, various components for the prescription including pharmaceutically acceptable common carrier, binder, stabilizer, excipient, diluent, pH buffer, disintegrant, solubilizer, solubilizing adjuvant and isotonic agent can be added. In addition, the above-described well-known substances having an effect of promoting osteogenesis and/or suppressing bone resorption, and minerals such as calcium, magnesium and phosphorus can also be used in combination. It is preferred that these preventative/therapeutic agents are orally administered in an ordinary administration form such as powder, granules, tablets, capsules, syrup or suspension. The dosage can be suitably selected depending on the purpose of the administration (prevention or treatment), kind and seriousness of the bone disease and age, etc. of a patient.

A method for manufacturing a functional food or food material for preventing/treating bone diseases of the present invention can be exemplified by a method of adding separately β-cryptoxanthin and zinc compound to food or food material, or a method of adding a composition containing β-cryptoxanthin and zinc compound, for example a composition for promoting osteogenesis or increasing bone mineral content of the present invention. The kinds of a food and food material having a function of preventing or treating bone diseases of the present invention, which are used for preventing/treating bone diseases, are not particularly limited. Examples include various drinks such as yogurt, yogurt drink, juice, cow's milk, soybean milk, liquor, coffee, black tea, green tea, oolong tea and sport drink; baked cakes such as puddings, cookies, breads, cakes, and rice crackers; Japanese cakes such as sweetened and jellied bean pastes; breads and cakes such as frozen sweets, jellies and chewing gums; noodles such as wheat noodles and buckwheat noodles; fish paste products such as steamed fish pastes, hams and fish sausages; seasonings such as *miso* (fermented soybean paste), soy sauce, dressings, mayonnaise and sweetening agents; milk products such as cheeses and butters; and various side dishes such as bean curds, *konnyaku* (a gelatinous food made from devil's-tongue starch) as well as *tsukudani* (some foods boiled in sweetened soy sauce), gyoza (dumplings stuffed with minced pork), croquettes and salads, honey, and royal jellies. These foods and food materials may further contain the above-described well-known materials having an osteogenesis promoting effect and/or bone resorption inhibiting effect, as well as minerals such as calcium, magnesium and phosphorus.

A method for preparing a feed composition of the present invention can be exemplified by a method of compounding β-cryptoxanthin and zinc compound separately, or a method of adding a composition containing β-cryptoxanthin and zinc compound, for example a composition for promoting osteogenesis or increasing bone mineral content of the present invention as a feed compounded component. A feed composition in which β-cryptoxanthin and zinc compound are compounded, can be used advantageously for growing domestic animals and poultry such as pigs, cattle and chickens; pets such as dogs and cats; and farmed fish and shellfish. Such feed composition may also contain the above-described well-known materials having an osteogenesis promoting effect and/or bone resorption inhibiting effect such as ipriflavones, as well as minerals such as calcium, magnesium, phosphorus, iron, zinc, manganese and copper. It is possible to indicate that it is effective for preventing or treating bone diseases, for example on the package or attached manual of the feed composition of the present invention.

The results in bone tissue culture system shown in the following examples are not exhibited only in a bone tissue culture system, empirically, but also when administered orally to a living body.

In the following examples, the present invention will be explained specifically, while the technical scope of the present invention is not limited to these exemplifications.

### Example 1

### (Determination of calcium level in bone tissues in a bone tissue culture system)

Rats (Wistar male rats; 4 weeks old, body weight 85 - 90 g; purchased from Japan SLC Ltd., and fed with solid Oriental yeast (MF)) were sacrificed under mild ether anesthesia. Muscle tissues around femur were removed completely, divided into diaphysis (cortical bone) and metaphysis (cancellous bone) tissues of femur, and then the bone marrow cells were washed and removed in a cold 0.25 M sucrose solution. The bone tissue pieces were cultured in a Dulbecco's modified Eagle's culture medium (serum-free, containing 50 units/ml penicillin and 50 µg/ml spretomycin, supplemented with 45 mg/ml glucose), in an CO₂ incubator aseptically for 48 hours at 37°C. In the culture solution, control (additive-free), β-cryptoxanthin (synthetic compound, 100% pure, 10⁻⁹ M), zinc (zinc sulfate, 10⁻⁶ M) and a mixed solution of β-cryptoxanthin (10⁻⁹ M) and zinc were added. Bone tissues were put into a culture dish (with a diameter of 35 mm, made of plastic) added with 2.0 ml of the above mentioned culture solution, and cultured. After being cultured, bone tissues were washed well in a cold 0.25 M sucrose solution, dried at 100°C for 5 hours, and weighed. Dried bone tissues were put into a test tube (15 ml), to which 2.0 ml of concentrated nitric acid was added, and the resultant was heated and degraded at 100°C for 16 hours. After degradation, solution amount was measured. A specific amount thereof was diluted with purified distilled water, and calcium was determined by colorimetry (kit for measuring calcium, Wako Pure Chemical Industries). Calcium levels in bone tissues are indicated as mg per 1 g of dried weight of cultured bone tissues. In the above experiment, bone tissues obtained from one rat were put in one culture dish, and 5 culture dishes were used for 5 rats. 5 dishes were used for each of the control, β-cryptoxanthin alone group, zinc alone group, and the β-cryptoxanthin and zinc-combined group. The results are shown by the mean level obtained from a culture of 5 dishes with 5 rats in each group, ± standard deviation, and statistically analyzed by Student's t test. A critical value of less than 5% was determined as having a significant difference.

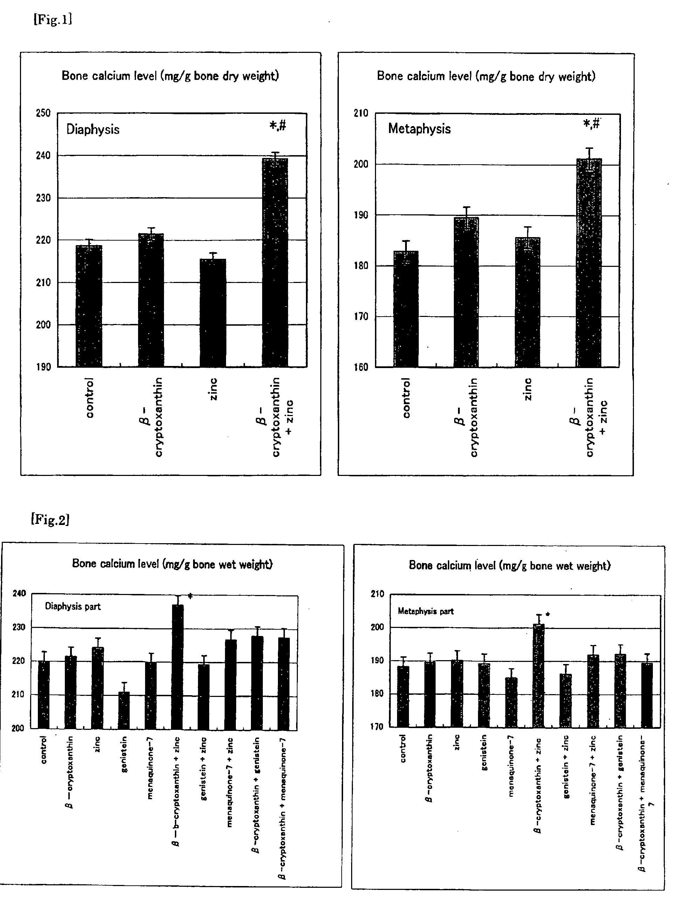

The results of culturing the above diaphysis (cortical bone) or metaphysis tissues of rat femur for 48 hours in a culture solution containing only β-cryptoxanthin (10⁻⁹ M), only zinc (10⁻⁶ M) and both β-cryptoxanthin (10⁻⁹ M) and zinc (10⁻⁶ M) are shown in Table 1 and Fig. 1.

**[Table 1]**

| Expression of synergetic effect enhancing calcium level in bone tissues by supplying β-cryptoxanthin and zinc | | |
|---|---|---|
| Administered group | Calcium level in bone tissues (mg/g bone dry weight) | |
| | diaphysis part | metaphysis part |
| Control | 218.7 ± 0.75 | 182.8 ± 2.52 |
| β-cryptoxanthin (10⁻⁹M) | 221.5 ± 3.95 | 189.5 ± 2.39 |
| zinc (10⁻⁶M) | 215.5 ± 2.01 | 185.6 ± 1.56 |
| β-cryptoxanthin (10⁻⁹M) + zinc (10⁻⁶M) | 239.3 ± 2.72 *# | 201.2 ± 2.14 *# |

| | | |
|---|---|---|
| Each level was obtained from bone tissues of 5 rats, and shows mean level ± standard deviation. * p < 0.01; compared with the control group (Student's t-test) # p > 0.01; compared with the level obtained with β-cryptoxanthin or zinc, alone (Student's t-test) | | |

As it is shown in Table 1 and Fig. 1, calcium levels in bone tissues both in diaphysis and metaphysis tissues did not change significantly in the group added with only β-cryptoxanthin (10⁻⁹ M) nor in the group added with only zinc (10⁻⁶M) compared to the control group (additive-free) . However a significant increase was observed in the group added with both β-cryptoxanthin (10⁻⁹M) and zinc (10⁻⁶ M).

### Example 2

### (Determination of calcium levels in bone tissues in a bone tissue culture system)

In order to investigate the effect on calcium levels in bone tissues when β-cryptoxanthin and zinc, or genistein and menaquinone-7 are supplied in the bone tissue culture system, femur diaphysis and metaphysis tissues were cultured for 48 hours in a culture solution, in the same manner as in Example 1, and the calcium level in bone tissues was measured, compared and evaluated. The results are shown in Table 2 and Fig. 2. Calcium levels in bone tissues did not increase significantly in the presence of β-cryptoxanthin (10⁻⁹ M), zinc (10⁻⁶M), genistein (10⁻⁶ M) and menaquinone-7 (10⁻⁶ M). With a higher concentration of 10⁻⁵ M, genistein and menaquinone-7 exhibited an effect of increasing significantly the calcium level in bone tissues, while no significant effect was observed when used separately in an amount of 10⁻⁶ M, respectively. Further, when β-cryptoxanthin (10⁻⁹ M) and zinc (10⁻⁶ M) were supplied, calcium levels in bone tissues increased significantly. Such effect was not observed when genistein (10⁻⁶ M) or menaquinone-7 (10⁻⁶ M) was added to β-cryptoxanthin (10⁻⁹ M), or when genistein (10⁻⁶ M) or menaquinone-7 (10⁻⁶ M) was added to zinc (10⁻⁶ M) . The calcium level in bone tissues did not increase significantly with a combination other than β-cryptoxanthin and zinc. Thus, it was found that the combination of β-cryptoxanthin (10⁻⁹ M) and zinc (10⁻⁶M) exhibits a further effective synergetic effect.

**[Table 2]**

| Change of calcium level in bone tissues by supplying β-cryptoxanthin, genistein, and menaquinone-7 | | |
|---|---|---|
| Administered group | Calcium level in bone tissues (mg/g bone dry weight) | |
| | diaphysis part | metaphysis part |
| Control | 220.0 ± 3.44 | 188.3 ± 3.65 |
| β-cryptoxanthin (10⁻⁹ M) | 221.5 ± 3.45 | 189.5 ± 3.44 |
| zinc(10⁻⁶M) | 224.3 ± 2.90 | 190.2 ± 2.53 |
| genistein (10⁻⁶M) | 211.0 ± 7.93 | 189.3 ± 6.40 |
| menaquinone-7 (10⁻⁶ M) | 219.8 ± 4.20 | 184.9 ± 2.70 |
| β-crypioxanthin (10⁻⁹M) + zinc (10⁻⁶M) | 237.0 ± 3.13* | 201.2 ± 2.40* |
| genistein (10⁻⁶ M) + zinc (10⁻⁶ M) | 219.3 ± 2.86 | 186.2 ± 4.03 |
| menaquinone-7 (10⁻⁶ M) + zinc (10⁻⁶ M) | 226.8 ± 5.30 | 192.0 ± 6.88 |
| β-cryptoxanthin (10⁻⁹ M) + genistein (10⁻⁶ M) | 227.8 ± 1.41 | 192.2 ± 3.82 |
| β-cryptoxanthin (10⁻⁹ M) + menaquinone-7 (10⁻⁶ M) | 227.4 ± 2.36 | 189.5 ± 1.84 |

| | | |
|---|---|---|
| Each level was obtained from bone tissues of 5 rats, and shows mean level ± standard deviation. * p < 0.01; compared with the control group, or with the level obtained with β-cryptoxanthin or zinc, alone (Student' s t-test) | | |

### Example 3

### (Determination of alkaline phosphatase activity in bone tissues in a bone tissue culture system)

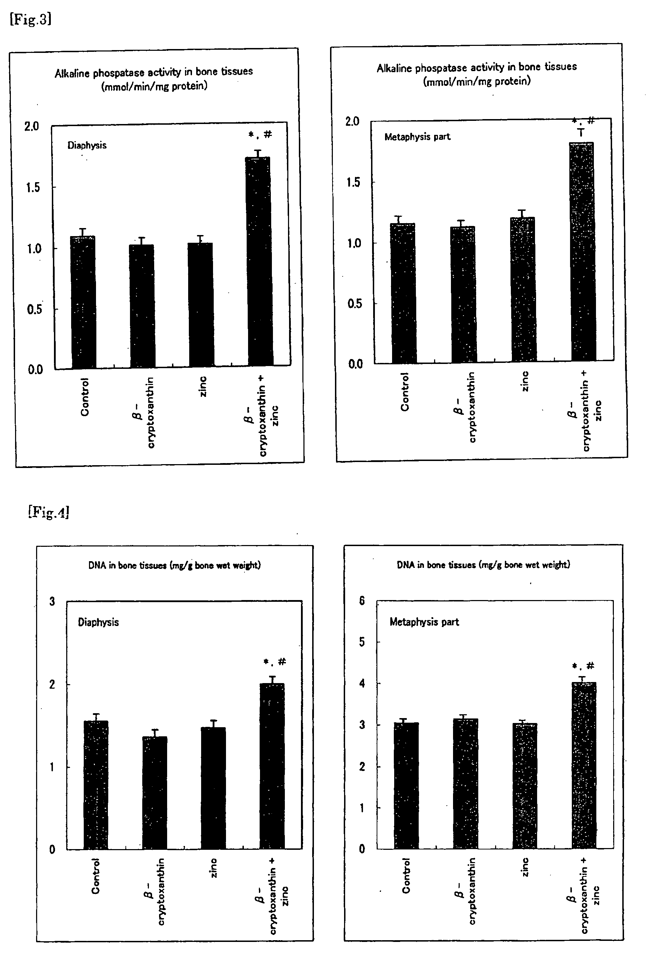

The expression level of alkaline phosphatase, which is the most important enzyme for promoting bone calcification, was examined. After culturing bone tissues in an incubator in the same manner as in Example 1, tissue pieces were washed in 0.25 M sucrose solution, then pulverized in 3 ml of 6.5 mM barbital buffer (pH 7.4) and treated with ultrasonic waves. The resultant liquid was centrifuged. The supernatant, as an enzyme solution, was determined by a method of Walter and Schutt (in Method of Enzymatic Analysis, Vol. 1-2, p. 856, Academic Press, New York, 1965). Namely, this method was carried out as follows: p-nitrophenylphosphoric acid was used as a substrate; 0.05 ml of the enzyme solution was added to 2 ml of diethanolamine buffer (pH 9.8). After the incubation at 37°C for 30 minutes, 10 ml of 0.05 N NaOH was added to the mixture. The absorbance (405 nm) was determined with a spectrophotometer to examine bone alkaline phosphatase activity of a therapeutic agent for bones and of a compound known to be effective for bones. The results are shown in Table 3 and Fig. 3.

**[Table 3]**

| Expression of synergetic effect enhancing alkaline phosphatase activity in bone tissues by supplying β-cryptoxanthin and zinc | | |
|---|---|---|
| Administered group | Alkaline phosphatase activity in bone tissues (µmol/min/mg protein) | |
| | diaphysis part | metaphysis part |
| Control | 1.094 ± 0.087 | 1.156 ± 0.099 |
| β-cryptoxanthin (10⁻⁹M) | 1.010 ± 0.078 | 1.122 ± 0.078 |
| zinc(10⁻⁶M) | 1.019 ± 0.061 | 1.190 ± 0.079 |
| β-cryptoxanthin (10⁻⁹M) + zinc (10⁻⁶M) | 1.712 ± 0.099 *# | 1.801 ± 0.123 *# |

| | | |
|---|---|---|
| Each level was obtained from bone tissues of 5 rats, and shows mean level ± standard deviation. * p < 0.01; compared with the control group (Student' s t-test) # p > 0.01; compared with the level obtained with β-cryptoxanthin or zinc, alone (Student's t-test) | | |

The alkaline phosphatase activity in bone tissues in a bone tissue culture system did not change significantly in the group added with only β-cryptoxanthin (10⁻⁹ M) nor in the group added with only zinc (10⁻⁶ M) compared to the control group, which is additive-free. However, when zinc (10⁻⁶ M) was supplied to β-cryptoxanthin (10⁻⁹ M), it was confirmed that the alkaline phosphatase activity in diaphysis and metaphysis tissues was significantly enhanced in a synergistic-manner. The synergetic effect was not observed in the presence of a protein synthetic inhibitor (cyclohexylimide 10⁻⁶ M)(results not shown), and it was found out that the above-mentioned synergetic effect is induced by the enhancement of protein synthesis in osteoblast.

### Example 4

### (Determination of DNA level in bone tissues in a bone tissue culture system)

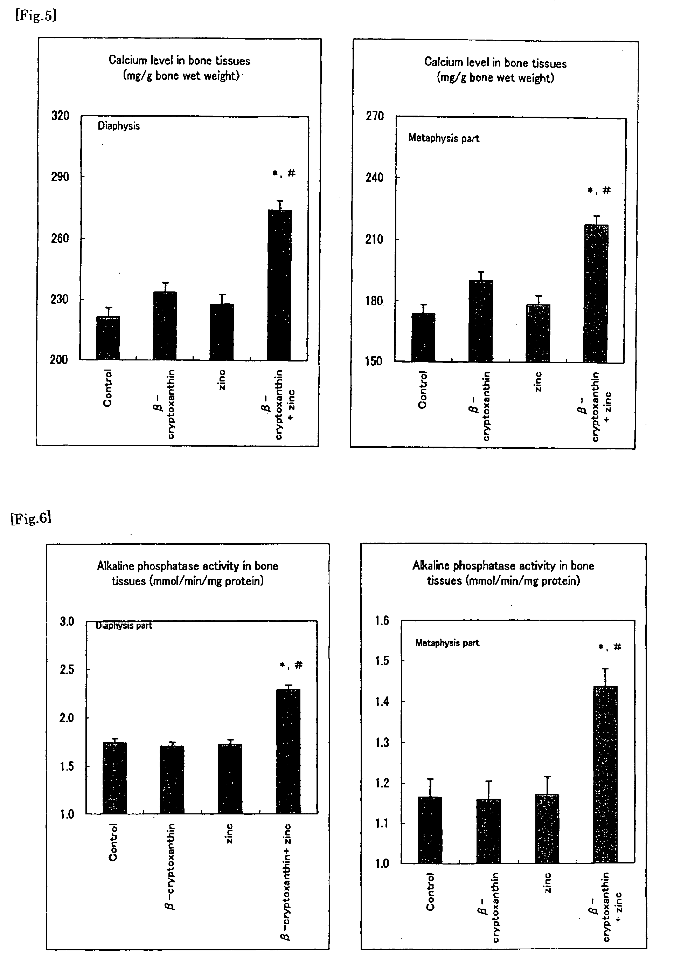

DNA level was determined as an index of cell count in bone tissues. After culturing bone tissues in an incubator in the same manner as in Example 1, tissue pieces were washed with 0.25 M sucrose solution to determine the wet weight, and then pulverized in 4 ml of 0.1 N NaOH. After osmosis at 4°C for 24 hours, the liquid mixture was centrifuged. The supernatant was taken as a sample and determined by a method of Ceriotti et al. (J. Biol. Chem., 241; 34-77, 1951). Namely, 1 ml of concentrated hydrochloric acid and 1 ml of 0.04 % indole solution were added to 2 ml of the sample and then the resultant mixture was heated to 100°C in boiling water. After quenching followed by an extraction with 4 ml of chloroform, the chloroform layer was taken to determine bone DNA level with a spectrophotometer (490 nm) . The results are shown in Table 4 and Fig. 4.

**[Table 4]**

| Expression of synergetic effect enhancing DNA level in bone tissues by supplying β-cryptoxanthin and zinc | | |
|---|---|---|
| Administered group | DNA level in bone tissues (mg/g bone wet weight) | |
| | diaphysis part | metaphysis part |
| Control | 1.551 ± 0.086 | 3.041 ± 0.113 |
| β-cryptoxanthin (10⁻⁹M) | 1.358 ± 0.089 | 3.141 ± 0.078 |
| zinc(10⁻⁶M) | 1.471 ± 0.052 | 3.025 ± 0.271 |
| β-cryptoxanthin(10⁻⁹M) + zinc (10⁻⁶M) | 2.000 ± 0.122 *# | 4.008 ± 0.170 *# |

| | | |
|---|---|---|
| Each level was obtained from bone tissues of 5 rats, and shows mean level ± standard deviation. * p < 0.01; compared with the control group (Student's t-test) # p > 0.01; compared with the level obtained with β-cryptoxanthin or zinc, alone (Student's t-test) | | |

DNA level in bone tissues in a bone tissue culture system did not change significantly in the group added with only β-cryptoxanthin (10⁻⁹ M) nor in the group added with only zinc (10⁻⁶ M) compared to the control group, which was additive free. However, when zinc (10⁻⁶ M) was supplied to β-cryptoxanthin (10⁻⁹ M), it was confirmed that the DNA level in diaphysis and metaphysis tissues was enhanced in a synergistic-manner. The synergetic effect was not observed in the presence of a protein synthetic inhibitor (cyclohexylimide 10⁻⁶M) (results not shown), and it was found out that the above-mentioned synergetic effect is induced by the enhancement of protein synthesis in osteoblast.

### Example 5

### (Determination of calcium level in bone tissues by oral administration)

Single administration of β-cryptoxanthin (5 µg/100 g body weight), single administration of zinc (0.1 mg/100 g body weight), and a combined administration of β-cryptoxanthin (5 µg/100 g body weight) and zinc (0.1 mg/100 g body weight) were performed by an oral administration with a feeding tube once a day for 7 days to rats (Wistar male rats; 4 to 5 weeks old; 85 - 90 g body weight) (purchased from Japan SLC Ltd., and fed with solid Oriental yeast (MF)). 24 hours after the final administration, femur was extracted, and the change in calcium levels in diaphysis and metaphysis tissues was examined with the method of Example 1. The results are shown in Table 5 and Fig. 5.

**[Table 5]**

| Expression of synergetic effect enhancing calcium level in bone tissues by oral administration supplying β-cryptoxanthin and zinc | | |
|---|---|---|
| Administered group | calcium level in bone tissues (mg/g dry mass) | |
| | diaphysis part | Metaphysis part |
| Control | 221.3 ± 4. 5 | 173 . 9 ± 5 . 9 |
| β-cryptoxanthin (5µg/100g body weight) | 233 . 6 ± 6. 8 | 190 . 1 ± 3. 4 |
| zinc(0.1 mg/100 g body weight) | 227.9 ± 4.3 | 178.6 ± 4 . 0 |
| β-cryptoxanthin (5µg/100g body weight) + zinc(0.1 mg/100 g body weight) | 274.0 ± 3.2 *# | 217.9 ± 3. 6 *# |

| | | |
|---|---|---|
| Each level was obtained from bone tissues of 5 rats, and shows mean level ± standard deviation. * p < 0.01; compared with the control group (Student's t-test) # p > 0.01; compared with the level obtained with β-cryptoxanthin or zinc, alone (Student's t-test) | | |

Oral administration of β-cryptoxanthin (5 µg/100 g body weight) alone did not induce a significant change of calcium level in diaphysis. However, the calcium level of methaphysis increased significantly. Further, oral administration of zinc (0.1 mg/100 g body weight) alone did not induce a significant change of calcium level either in diaphysis or in metaphysis tissues. On the other hand, when zinc (0.1 mg/100 g body weight) was supplied to β-cryptoxanthin (5 µg/100 g body weight) and administered orally for 7 days, the calcium level in diaphysis and metaphysis tissues increased in a synergistic-manner, compared to when β-cryptoxanthin and zinc were administered alone. This result was observed when it was administered orally to rats, similarly as in the bone tissue culture system.

### Example 6

### (Determination of alkaline phosphatase activity in bone tissues by oral administration)

The change of alkaline phosphatase activity in diaphysis and metaphysis tissues which were extracted in the same manner as in Example 5 was examined with the method of Example 3. The results are shown in Table 6 and Fig. 6. As a result, oral administration of β-cryptoxanthin (5 µg/100 g body weight) alone did not induce a significant change of alkaline phosphatase activity in diaphysis and metaphysis tissues. Oral administration of zinc (0.1 mg/100 g body weight) alone did not induce a significant change of alkaline phosphatase activity in diaphysis and metaphysis tissues. On the other hand, when zinc (0.1 mg/100 g body weight) was supplied to β-cryptoxanthin (5 µg/100 g body weight) and administered orally for 7 days, the alkaline phosphatase activity in diaphysis and metaphysis tissues increased in a synergistic-manner, compared to when β-cryptoxanthin and zinc were administered alone. This result was observed when it was administered orally to rats, similarly as in the bone tissue culture system.

**[Table 6]**

| Expression of synergetic effect enhancing alkaline phosphatase activity in bone tissues by oral administration supplying β-cryptoxanthin and zinc | | |
|---|---|---|
| Administered group | Alkaline phosphatase activity in bone tissues (µmol/min/mg protein) (dry mass) | |
| | diaphysis part | metaphysis part |
| Control | 1.739 ± 0.045 | 1.165 ± 0.051 |
| β-cryptoxanthin (5µg/100g body weight) | 1.703 ± 0.056 | 1.159 ± 0.048 |
| zinc(0.1 mg/100 g body weight) | 1.725 ± 0.038 | 1.170 ± 0.033 |
| β-cryptoxanthin (5µg/100g body weight) + zinc(0.1 mg/100 g body weight) | 2.289 ± 0.040 *# | 1.435 ± 0.049 *# |

| | | |
|---|---|---|
| Each level was obtained from bone tissues of 5 rats, and shows mean level ± standard deviation. * p < 0.01; compared with the control group (Student's t-test) # p > 0.01; compared with the level obtained with β-cryptoxanthin or zinc, alone (Student's t-test) | | |

### Example 7

### (Determination of DNA level in bone tissues by oral administration)

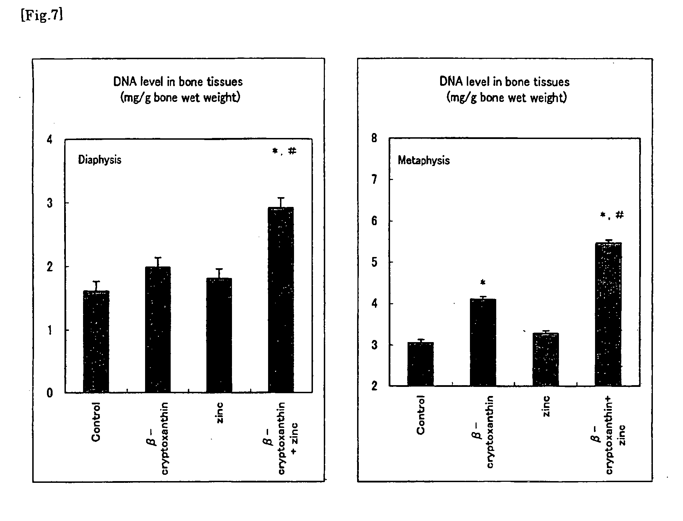

The change of DNA level in diaphysis and metaphysis tissues which were extracted in the same manner as in Example 5 was examined with the method of Example 4. The results are shown in Table 7 and Fig. 7. As a result, oral administration of β-cryptoxanthin (5 µg/100 g body weight) alone did not induce a significant change of DNA level in diaphysis, but lead to a significant increase in metaphysis tissues. Oral administration of zinc (0.1 mg/100 g body weight) alone did not induce a significant change of DNA level in diaphysis and metaphysis tissues. On the other hand, when zinc (0.1 mg/100 g body weight) was supplied to β-cryptoxanthin (5 µg/100 g body weight) and administered orally for 7 days, the DNA level in diaphysis and metaphysis tissues increased in a synergistic-manner, compared to when β-cryptoxanthin and zinc were administered alone. This result was observed when it was administered orally to rats, similarly as in the bone tissue culture system.

**[Table 7]**

| Expression of synergetic effect enhancing DNA level in bone tissues by oral administration supplying β-cryptoxanthin and zinc | | |
|---|---|---|
| Administered group | DNA level in bone tissues (mg/g bone wet weight) (mg/g dry mass) | |
| | diaphysis part | metaphysis part |
| Control | 1.615 ± 0.048 8 | 3.059 ± 0.041 |
| β-cryptoxanthin (5µg/100g body weight) | 1.980 ± 0.251 | 4.102 ± 0.055* |
| zinc(0.1 mg/100 g body weight) | 1.805 ± 0.154 | 3.278 ± 0.103 |
| β-cryptoxanthin (5µg/100g body weight) + zinc(0.1 mg/100 g body weight) | 2.915 ± 0.151 *# | 5.468 ± 0.100 *# |

| | | |
|---|---|---|
| Each level was obtained from bone tissues of 5 rats, and shows mean level ± standard deviation. * p < 0.01; compared with the control group (Student's t-test) # p > 0.01; compared with the level obtained with β-cryptoxanthin or zinc, alone (Student's t-test) | | |

Further, when administering a combination of β-cryptoxanthin (10 µg/100 g body weight) and zinc (0.5 µg/100 g body weight), it was confirmed that a synergetic effect of bone components (calcium level, alkaline phosphatase activity, and DNA level) was exhibited (results not shown). From these results, according to the present invention, by combining β-cryptoxanthin and zinc in a concentration that does not exhibit an effect of increasing the calcium level, alkaline phosphatase activity and DNA level in bone tissues when used separately, an effect of exhibiting a significant increase of the calcium level, alkaline phosphatase activity and DNA level in bone tissues was observed, both in vivo and in vitro, in a synergistic-manner.

### Industrial Applicability

According to the present invention, by using β-cryptoxanthin and zinc compound in an amount that is not effective when used separately, an osteogenesis promoter having a significant effect that can prevent/treat bone diseases by promoting actively osteogenesis, and a food and drink, pharmaceuticals, or feed which is useful for preventing/treating bone diseases such as osteoporosis having both osteogenesis promoting effect and bone resorption inhibiting effect can be provided.

## Claims

1. A composition for promoting osteogenesis and increasing bone mineral content comprising β-cryptoxanthin and zinc compound as active ingredients.

2. The composition for promoting osteogenesis and increasing bone mineral content according to claim 1, wherein β-cryptoxanthin and zinc compound are contained at a concentration that does not exhibit an effect of increasing calcium level in bone tissues when used independently.

3. A preventative/therapeutic agent for bone diseases comprising β-cryptoxanthin and zinc compound as active ingredients.

4. The preventative/therapeutic agent for bone diseases according to claim 3, wherein β-cryptoxanthin and zinc compound are contained at a concentration that does not exhibit an effect of increasing calcium level in bone tissue when used independently.

5. A preventative/therapeutic agent for bone diseases according to claim 4, wherein the bone disease is osteoporosis.

6. A functional food or food material for preventing/treating bone diseases, wherein β-cryptoxanthin and zinc compound are added.

7. The functional food or food material for preventing/treating bone diseases according to claim 6, wherein β-cryptoxanthin and zinc compound are contained at a concentration that does not exhibit an effect of increasing calcium level in bone tissue when used independently.

8. A food and drink according to claim 7, wherein the bone disease is osteoporosis.

9. A feed composition wherein β-cryptoxanthin and zinc compound are compounded.

10. The feed composition according to claim 9, wherein β-cryptoxanthin and zinc compound are contained at a concentration that does not exhibit an effect of increasing calcium level in bone tissues when used independently.
